(19) Europäisches Patentamt
European Patent Office
Office européen des brevets

(11) Numéro de publication : **0 015 625**
**B1**

(12) # FASCICULE DE BREVET EUROPÉEN

(45) Date de publication du fascicule du brevet : **22.09.82**

(51) Int. Cl.$^3$ : **C 07 C 17/38**

(21) Numéro de dépôt : **80200208.9**

(22) Date de dépôt : **05.03.80**

(54) **Procédé pour le traitement de produits lourds résultant de la fabrication d'hydrocarbures chlorés.**

(30) Priorité : **12.03.79 FR 7906674**

(43) Date de publication de la demande : **17.09.80 (Bulletin 80/19)**

(45) Mention de la délivrance du brevet : **22.09.82 Bulletin 82/38**

(84) Etats contractants désignés : **AT BE CH DE FR GB IT LU NL SE**

(56) Documents cités :
**DE A 2 053 632**
**US A 2 356 785**

(73) Titulaire : **SOLVAY & Cie (Société Anonyme)**
**Rue du Prince Albert, 33**
**B-1050 Bruxelles (BE)**

(72) Inventeur : **Hembersin, Roland**
**Route d'Eghezée, 120**
**B-5790 Jemeppe-sur-Sambre (BE)**
Inventeur : **Nicaise, Remy**
**Rue de Châtelet, 148**
**B-6290 Nalinnes (BE)**

(74) Mandataire : **Elschen, Roland**
**Solvay & Cie Département de la Propriété Industrielle**
**Rue de Ransbeek 310**
**B-1120 Bruxelles (BE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

EP 0 015 625 B1

Procédé pour le traitement de produits lourds résultant de la fabrication d'hydrocarbures chlorés

La présente invention concerne un procédé pour le traitement de produits lourds résultant de la fabrication d'hydrocarbures chlorés et, plus particulièrement, le traitement des produits lourds résultant de la fabrication du 1,2-dichloréthane, du trichloréthylène, du perchloréthylène, des trichloréthanes ou du tétrachlorométhane.

Lors de la fabrication des hydrocarbures chlorés, il se forme des produits lourds de composition mal définie appelés généralement « goudrons ». Ces produits, qui sont constitués principalement d'hydrocarbures chlorés à haut point d'ébullition se retrouvent dans les bouilleurs des colonnes à distiller utilisées pour la séparation et la purification des produits. En général, on les élimine en procédant à une purge du contenu des bouilleurs mais on provoque ainsi des pertes importantes en hydrocarbures chlorés valorisables.

Diverses techniques ont été développées pour essayer de récupérer les hydrocarbures chlorés valorisables présents dans les purges.

Ainsi, on a proposé, dans le brevet belge 746 200 déposé le 19 février 1970 au nom de STAUFFER CHEM. CO, d'envoyer, lors de la fabrication du 1,2-dichloréthane, les purges des bouilleurs de la colonne à 1,2-dichloréthane dans des évaporateurs à film où l'on récupère en tête un mélange de 1,2-dichloréthane et de 1,1,2-trichloréthane et en pied un mélange de produits chlorés lourds dont la séparation est techniquement et économiquement sans intérêt et qui est brûlé. Cette technique est très difficile à mettre en œuvre dans une installation industrielle fonctionnant en continu. En effet, la conduite d'un évaporateur à film est très délicate et il faut arrêter fréquemment l'installation pour procéder au nettoyage des dépôts et des croûtes. En outre, pour éviter un encrassement trop rapide du pied de l'évaporateur, il faut limiter le taux de vaporisation à un pourcentage faible, ce qui ne permet pas de récupérer les produits valorisables dans leur totalité.

Par ailleurs, on a essayé de mettre en œuvre un procédé de distillation par entraînement à la vapeur d'eau. Pareil procédé permet effectivement de récupérer en tête les produits légers récupérables mais les produits lourds restant en pied forment avec l'eau une émulsion qui reste stable même quand on la soumet à une centrifugation. Etant donné que l'émulsion obtenue, qui ne peut être brûlée du fait de sa teneur en eau élevée et qui n'est pas recyclable, ne peut être évacuée telle quelle vers l'environnement, le procédé de distillation par entraînement à la vapeur d'eau était inutilisable.

On a maintenant trouvé un procédé qui ne présente pas les inconvénients du procédé où l'on utilise un évaporateur à film et qui rend la distillation par entraînement à la vapeur utilisable. Ce procédé permet de récupérer, en tête, l'hydrocarbure chloré souhaité et les sous-produits de chloration valorisables et, en pied, une phase aqueuse ainsi qu'une phase organique contenant les produits lourds non récupérables parfaitement séparée de la phase aqueuse.

La présente invention concerne à cet effet un procédé pour le traitement des produits lourds résultant de la fabrication d'hydrocarbures chlorés, selon lequel on soumet les produits lourds à une distillation par entraînement à la vapeur d'eau en présence d'un agent tensioactif dont la solubilité dans l'eau est inférieure à 1 g/l lorsque l'eau est en équilibre avec une solution à 10 g/l d'agent tensioactif dans le 1,2-dichloroéthane à 20 °C.

Les agents tensioactifs mis en œuvre selon l'invention sont habituellement solubles dans la phase organique contenant les produits lourds. De préférence, on choisit les agents tensioactifs parmi ceux qui sont chimiquement inertes dans les conditions acides ou alcalines dans lesquelles on réalise le procédé. En outre, on les choisit de préférence parmi ceux qui ne contiennent pas de produits légers susceptibles de contaminer la phase de tête des produits organiques récupérables. Enfin, on choisit de préférence ceux dont la température d'ébullition est suffisamment élevée pour qu'ils restent dans la phase organique lourde récupérée en pied. Les agents tensioactifs préférés ont, dans les conditions précitées, une solubilité inférieure à 0,8 g/l. Les meilleurs résultats ont été obtenus avec des agents dont la solubilité telle que définie ci-avant est inférieure à 0,5 g/l.

Le choix d'agents tensioactifs non solubles dans l'eau est une caractéristique essentielle de l'invention. En effet, on a constaté que les autres agents tensioactifs provoquent la formation d'émulsions eau-produits chlorés lourds qui sont stables, voire même plus stables que les émulsions ne contenant pas d'agents tensioactifs.

On choisit habituellement les agents tensioactifs utilisés selon l'invention parmi ceux dont la balance hydrophile-lipophile, (HLB) telle que définie par P. Blonchard dans PARF. COSM. SAV., Vol. 12 n° 2 de février 1969, p. 82-91, et exprimée par l'équation

$$HLB = \frac{1 \times \text{masse moléculaire de la partie hydrophile} \times 100}{5 \times \text{masse moléculaire totale}}$$

est au plus égale à 9. De préférence, les agents tensioactifs utilisés selon l'invention ont un HLB compris entre 4 et 7. Les meilleurs résultats ont été obtenus avec les agents tensioactifs dont le HLB est compris entre 5 et 6.

Les agents tensioactifs utilisés sont choisis le plus souvent parmi les amines grasses, les alcools

gras, les alkylphénols, les acides gras, les amides d'acides gras mono- ou polyéthoxylés, les esters d'acides gras, les condensats d'oxyde d'éthylène et d'oxyde de propylène et les éthanolamides.

Parmi les agents tensioactifs énumérés ci-dessus les amines grasses éthoxylées conviennent particulièrement bien et les meilleurs résultats ont été obtenus avec les amines grasses mono-, di- et tri-éthoxylées. Plus particulièrement, on préfère travailler avec des amines dont la formule générale est

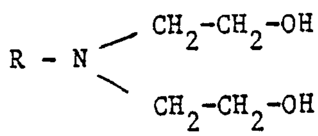

dans laquelle R représente des radicaux alkyles, saturés ou insaturés, ayant de 12 à 24 atomes de carbone. Les composés qui conviennent le mieux sont ceux dans lesquels R possède de 16 à 20 atomes de carbone. Les meilleurs résultats ont été obtenus avec des amines dans lesquelles R possède 18 atomes de carbone.

La quantité d'agent tensioactif à mettre en œuvre selon l'invention est choisie en tenant compte de l'origine et de la nature des produits chlorés lourds à traiter. En général, l'agent tensioactif est mis en œuvre à raison de 0,001 à 20 g/kg de produits lourds à traiter et, de préférence, à raison de 0,005 à 10 g/kg. Des résultats particulièrement avantageux ont été obtenus avec des doses allant de 0,01 à 5 g d'agent tensioactif par kg de produit chloré lourd à traiter. La mise en œuvre de quantités trop importantes d'agent tensioactif peut engendrer dans certaines circonstances des émulsions eau-produits chlorés lourds qui sont stables. Il convient, dans ce cas, de diminuer la dose d'agent tensioactif.

Il va de soi que plusieurs agents tensioactifs différents peuvent être mis en œuvre simultanément selon l'invention. Dans ce cas, on préfère cependant que l'indice HLB du mélange satisfasse aux conditions définies ci-avant et il convient que chacun des composés constituant le mélange ait une solubilité dans l'eau qui satisfait aux conditions de solubilité définies ci-avant.

L'agent tensioactif peut être introduit dans les produits lourds avant qu'ils sont soumis à la distillation par entraînement à la vapeur. Une façon de procéder particulièrement avantageuse consiste à introduire l'agent tensioactif directement dans les produits lourds à traiter avant ou pendant la distillation par entraînement à la vapeur d'eau. Ce dernier mode d'exécution permet de réaliser le procédé selon l'invention avec un minimum de manipulations et de pertes énergétiques.

L'agent tensioactif peut être introduit en continu ou en discontinu dans les produits lourds à traiter.

Les produits chlorés lourds traités selon l'invention sont des produits de composition mal définie appelés également « goudrons ». Ces produits sont généralement constitués d'un ensemble de sous-produits polychlorés à haut point d'ébullition, tous généralement présents dans des petites concentrations ce qui rend leur récupération et séparation difficile et généralement non rentable d'un point de vue industriel.

Les produits chlorés lourds que l'on peut traiter selon l'invention peuvent provenir d'origines diverses. Ils résultent en général de la fabrication ou de l'épuration de produits chlorés légers d'une même ou de différentes origines. On entend, par produits chlorés légers, les composés chlorés aromatiques contenant moins de 10 atomes et de préférence de 6 à 8 atomes de carbone, et les composés aliphatiques chlorés, saturés ou insaturés, à chaîne droite ou ramifiée, cyclisés ou non, contenant moins de 8 atomes et de préférence moins de six atomes de carbone.

Le procédé selon l'invention s'applique avantageusement aux produits chlorés lourds résultant de la fabrication de composés aliphatiques ayant moins de 4 atomes de carbone et plus particulièrement la fabrication du chlorure de vinyle, du 1,2-dichloréthane, du trichloréthylène, du perchloréthylène, des trichloréthanes et des chlorométhanes. De très bons résultats ont été obtenus lorsque le procédé est appliqué aux produits chlorés lourds résultant de la fabrication du 1,2-dichloréthane.

Les produits chlorés lourds peuvent, avant pendant ou après la distillation par entraînement à la vapeur être soumis à divers traitements chimiques ou physiques. Ainsi, on peut, par exemple, introduire dans les produits lourds en vue de la distillation par entraînement à la vapeur d'eau un composé basique afin d'éviter une acidification excessive du milieu due à la formation d'acide chlorhydrique généré par la décomposition de certains composés organiques chlorés instables dans les conditions opératoires.

La distillation par entraînement à la vapeur d'eau peut être effectuée par toute technique connue à cet effet. L'opération d'entraînement à la vapeur ne revêt en soi-même aucun caractère critique pour la présente invention. La température et la pression de la vapeur introduite dans les produits chlorés lourds sont choisies généralement en fonction de la nature de ceux-ci. Ces paramètres ne sont pas critiques en eux-mêmes et sont ceux habituellement utilisés dans les techniques d'entraînement à la vapeur d'eau.

L'entraînement à la vapeur est généralement conduit pendant une durée telle qu'au moins 20 % en volume des produits légers de départ sont entraînés et récupérés sous forme de produit de tête de distillation. Ce taux de distillation n'est pas critique en lui-même et peut varier suivant la composition des produits chlorés lourds traités. Ainsi si ceux-ci contiennent beaucoup de produits légers récupérables et valorisables, on choisit des taux de distillation plus élevés. Dans la récupération des sous-produits de la fabrication du 1,2-dichloréthane on travaille généralement avec des taux de distillation compris entre 25 %

et 85 % ; avantageusement les taux de distillation varient entre 35 % et 80 %.

La fraction de tête recueillie dans le procédé selon l'invention est constituée d'une fraction organique et d'une phase aqueuse distincte. La fraction organique est généralement constituée par le produit principal de la fabrication de départ et par les sous-produits légers principaux de cette fabrication. Ces produits organiques sont séparés finalement dans les colonnes de distillation du cycle de fabrication du produit chloré léger de départ auxquelles la fraction de tête est recyclée. La phase aqueuse recueillie en tête est pure à plus de 99 %.

La fraction de pied restant après l'opération d'entraînement à la vapeur d'eau est également constituée, grâce à l'adjonction de l'agent tensioactif, de deux phases bien distinctes. La phase organique est constituée de résidus chlorés lourds, non récupérables actuellement, qui peuvent être stockés en vue d'une utilisation éventuelle ultérieure ou qui peuvent être détruits par les moyens, habituellement utilisés à cet effet, tels que le brûlage en mer. La fraction aqueuse distincte récupérée en pied est tout comme la phase aqueuse recueillie avec la fraction de tête pure à plus de 99 %. Dès lors, on peut avantageusement réutiliser les phases aqueuses, recueillies en tête et en pied, dans un nouveau cycle d'entraînement à la vapeur.

Les exemples suivants sont donnés à titre non limitatif.

## Exemple 1

Dans un ballon Wülff de 1 litre on introduit 100 $cm^3$ (125 g) de produits chlorés lourds, résultant de la fabrication de 1,2-dichloréthane, dont la composition, déterminée par chromatographie en phase vapeur, est donnée au Tableau 1 et 0,5 g/l, de produits chlorés lourds mis en œuvre, d'agent tensioactif TENSOXID S20 vendu par la firme TENSIA et qui est un condensat d'oxyde d'éthylène sur amine de suif.

Le ballon est ensuite chauffé à 90 °C et on fait passer de la vapeur d'eau sous pression atmosphérique et en continu à travers le mélange de produits chlorés lourds suivant la technique bien connue de la distillation par entraînement à la vapeur d'eau. Le produit organique entraîné par la vapeur d'eau est condensé et récupéré dans une éprouvette graduée de 200 $cm^3$. La distillation est arrêtée quand l'éprouvette contient 75 $cm^3$ de produits chlorés entraînés (taux de distillation : 75 %). Les fractions organique et aqueuse distinctes recueillies en tête et les fractions organique et aqueuse distinctes recueillies en pied du ballon de 1 l sont ensuite analysées par chromatographie en phase vapeur et les résultats sont rassemblés au Tableau 1.

(Voir Tableau, p. 5)

TABLEAU 1

| Composés | Composition en produit lourd | | FRACTION DE TETE | | | | FRACTION DE PIED | | | |
|---|---|---|---|---|---|---|---|---|---|---|
| | | | Phase organique | | Phase aqueuse | | Phase organique | | Phase aqueuse | |
| | quantité g | teneur g/kg | quantité g | teneur g/kg | quantité g | teneur g/kg | quantité g | teneur g/kg | quantité g | teneur g/kg |
| 1,2-dichloréthane | 25,0 | 200 | 24,38 | 260 | 0,06 | 2,1 | 0,34 | 11,0 | 0,03 | 0,45 |
| 1,1,2-trichloréthane | 55,38 | 443 | 44,91 | 479 | 0,05 | 1,8 | 9,22 | 295 | 0,14 | 2,0 |
| 1,3-dichlorpropane | 1,23 | 9,8 | 0,76 | 8,1 | <0,01 | 0,02 | 0,12 | 3,7 | - | - |
| 3,4-dichlorbutène-1 | 0,48 | 3,8 | 0,34 | 3,6 | - | - | - | - | - | - |
| 1,2-dichlorbutane | 2,63 | 21 | 1,50 | 16,0 | <0,01 | 0,01 | 0,47 | 15,0 | <0,01 | 0,03 |
| Divers | 24,28 | 194,4 | 16,69 | 178,0 | <0,01 | 0,02 | 4,09 | 131 | 0,06 | 0,89 |
| Composés lourds non identifiés et identifiés | 16 | 128 | 5,18 | 55,2 | <0,01 | 0,15 | 17,0 | 544 | 0,20 | 5,4 |
| TOTAL | 125,0 | | 93,76 | | 0,12 | | 31,24 | | 0,43 | |

N.B. Les transformations de g/kg en g ont été effectuées en assimilant le poids spécifique des composés lourds à celui du 1,2-dichloréthane (1,25). Les composés divers sont constitués notamment de perchloréthylène, de 1,3-dichlorobutène cis, 1,3-dichlorobutène trans, 1,1,1,2-tétrachloréthane et 1,1,2-trichloropropane.

La phase aqueuse récupérée en tête contient également 0,03 g de produits plus légers que le 1,2-dichloréthane (chlorure de méthylène, 1,1-dichloréthane, chloroforme, 1,2-dichloréthylène cis et trans).

Exemple 2

On a répété l'exemple 1 mais en partant de produits chlorés lourds obtenus à partir d'une fabrication de 1,2-dichloréthane d'une autre origine et d'une teneur en agent tensioactif TENSOXID S20 de 1 g/l de produits chlorés lourds mis en œuvre.

Les conditions opératoires sont les mêmes que celles de l'exemple 1 à l'exception du taux de distillation qui a été limité à 50 %.

Les résultats obtenus sont rassemblés dans le Tableau 2 ci-dessous.

(Voir Tableau, p. 7)

TABLEAU 2

| Composés | Composition en produit lourd | | FRACTION DE TETE | | | | FRACTION DE PIED | | | |
|---|---|---|---|---|---|---|---|---|---|---|
| | | | Phase organique | | Phase aqueuse | | Phase organique | | Phase aqueuse | |
| | quantité g | teneur g/kg | quantité g | teneur g/kg | quantité g | teneur g/kg | quantité g | teneur g/kg | quantité g | teneur g/kg |
| Légers | 1,58 | 12,6 | 1,16 | 18,6 | - | - | 1,49 | 23,8 | 0,12 | 2,3 |
| 1,2-dichloréthane | 19,5 | 156 | 17,37 | 278 | 0,03 | 2,46 | 2,94 | 47 | 0,01 | 0,53 |
| Moyens | 0,25 | 2,0 | 0,15 | 2,5 | - | - | 0,11 | 1,7 | - | - |
| trichloréthylène | 0,11 | 0,9 | 0,04 | 0,7 | - | - | 0,06 | 1,0 | - | - |
| 1,1,2-trichloréthane | 65,6 | 525 | 33,1 | 530 | 0,04 | 2,74 | 31,9 | 511 | - | - |
| perchloréthylène | 8,75 | 70 | 4,81 | 77 | <0,01 | 0,02 | 3,69 | 59 | - | - |
| 1,1,1,2-tétrachlor-éthane | 6,75 | 54 | 2,19 | 35 | - | - | 4,06 | 65 | - | - |
| 1,1,2,2-tétrachlor-éthane | 5,5 | 44 | 1,38 | 22 | - | - | 3,75 | 60 | - | - |
| 1,4-dichlorobutane | 6,63 | 53 | 1,19 | 19 | - | - | 5,72 | 90 | - | - |
| Lourds | 10,15 | 81 | 1,19 | 19 | - | - | 8,81 | 141 | - | - |
| TOTAL | 125,0 | | 62,58 | | 0,07 | | 62,43 | | 0,13 | |

N.B. Les transformations de g/kg en g ont été effectuées en assimilant le poids spécifique des composés lourds à celui du 1,2-dichloréthane (1,25). Les composés légers sont des produits du type chloroforme et 1,1-dichloréthane. Les composés moyens et lourds sont des produits qui n'ont pas été identiiiés.

On peut déduire des résultats observés que la séparation en phases distinctes séparables est quasi parfaite et que la récupération en produits légers récupérables (1,2-dichloréthane et 1,1,2-trichloréthane) est respectivement de 99 % et de 80 % pour un taux de distillation de 75 % (exemple 1) ; ces valeurs atteignent encore 90 % et 55 % pour un taux de distillation ne dépassant pas 50 % (exemple 2). En outre, les phases aqueuses obtenues sont quasiment pures.

**Revendications**

1. Procédé pour le traitement des produits lourds résultant de la fabrication d'hydrocarbures chlorés selon lequel on soumet les produits lourds à une distillation par entraînement à la vapeur d'eau, caractérisé en ce que l'on opère la distillation en présence d'un agent tensioactif dont la solubilité dans l'eau est inférieure à 1 g/l lorsque l'eau est en équilibre avec une solution à 10 g/l d'agent tensioactif dans le 1,2-dichloroéthane à 20 °C.

2. Procédé selon la revendication 1, caractérisé en ce qu'il est appliqué à des produits lourds résultant de la fabrication du 1,2-dichloréthane.

3. Procédé selon les revendications 1 ou 2, caractérisé en ce que l'on utilise un agent tensioactif possédant un indice HLB compris entre 4 et 7.

4. Procédé selon l'une quelconque des revendications 1 à 3, caractérisé en ce que l'on utilise une amine grasse mono-, di- ou tri-éthoxylée.

5. Procédé selon la revendication 4, caractérisé en ce que l'on utilise une amine grasse éthoxylée de formule générale

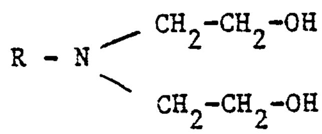

dans laquelle R représente un radical alkyle ayant de 16 à 20 atomes de carbone.

6. Procédé selon la revendication 5, caractérisé en ce que R est un radical alkyle ayant 18 atomes de carbone.

7. Procédé selon l'une quelconque des revendications 1 à 6, caractérisé en ce que l'on met en œuvre l'agent tensioactif à raison de 0,005 à 10 g/kg de produit lourd chloré traité.

**Claims**

1. Process for the treatment of the heavy products resulting from the manufacture of chlorinated hydrocarbons, in which the heavy products are subjected to steam distillation, characterised in that the distillation is carried out in the presence of a surface-active agent of which the solubility in water is less than 1 g/l when the water is in equilibrium with a solution containing 10 g/l of surface-active agent in 1,2-dichloroethane at 20 °C.

2. Process according to Claim 1, characterised in that it is applied to heavy products resulting from the manufacture of 1,2-dichloroethane.

3. Process according to Claims 1 or 2, characterised in that a surface-active agent having an HLB index of between 4 and 7 is used.

4. Process according to any one of Claims 1 to 3, characterised in that a mono-, di- or tri-oxyethyleneated fatty amine is used.

5. Process according to Claim 4, characterised in that an oxyethyleneated fatty amine of the general formula

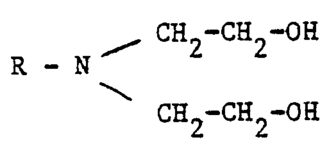

is used, in which R represents an alkyl radical having from 16 to 20 carbon atoms.

6. Process according to Claim 5, characterised in that R is an alkyl radical having 18 carbon atoms.

7. Process according to any one of Claims 1 to 6, characterised in that the surface-active agent is

used in an amount of 0.005 to 10 g/kg of chlorinated heavy product treated.

**Ansprüche**

1. Verfahren zur Behandlung von Schwerprodukten, wie sie bei der Herstellung von chlorierten Kohlenwasserstoffen anfallen, bei welchem man die Schwerprodukte einer Destillation mit Wasserdampf als Schleppmittel unterwirft, dadurch gekennzeichnet, daß man die Destillation in Anwesenheit eines oberflächenaktiven Mittels durchführt, dessen Löslichkeit in Wasser unterhalb von 1 g/l ist, wenn das Wasser im Gleichgewicht steht mit einer Lösung von 10 g/l des oberflächenaktiven Mittels in 1,2-Dichloräthan bei 20 °C.

2. Verfahren gemäß Anspruch 1, dadurch gekennzeichnet, daß es angewandt wird auf Schwerprodukte, wie sie bei der Herstellung von 1,2-Dichloräthan anfallen.

3. Verfahren gemäß Ansprüchen 1 oder 2, dadurch gekennzeichnet, daß man ein oberflächenaktives Mittel verwendet, welches einen HLB-Index von zwischen 4 und 7 besitzt.

4. Verfahren gemäß einem der Ansprüche 1 bis 3, dadurch gekennzeichnet, daß man ein mono-, di- oder tri-äthoxyliertes Fettamin benutzt.

5. Verfahren gemäß Anspruch 4, dadurch gekennzeichnet, daß man ein äthoxyliertes Fettamin der allgemeinen Formel

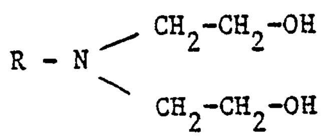

in welcher R einen Alkylrest mit 16 bis 20 Kohlenstoffatomen darstellt, benutzt.

6. Verfahren gemäß Anspruch 5, dadurch gekennzeichnet, daß R ein Alkylrest mit 18 Kohlenstoffatomen ist.

7. Verfahren gemäß einem der Ansprüche 1 bis 6 dadurch gekennzeichnet, daß man das oberflächenaktive Mittel in einer Menge von 0,005 bis 10 g/kg behandeltem chlorierten Schwerprodukt einsetzt.